# EUROPEAN PATENT SPECIFICATION

(11) **EP 0 823 416 B1**
(45) Date of publication and mention of the grant of the patent: **29.12.1999**
(21) Application number: 97305973.6
(22) Date of filing: 06.08.1997
(51) Int. Cl.: C07C 227/16, C07C 229/28

(54) **Process for producing cyclohexylamino acids**
Verfahren zur Herstellung von Cyclohexylaminosäuren
Procédé pour la préparation de cyclohexylamino acides

(30) Priority: 07.08.1996 JP 20802996
(43) Date of publication of application: 11.02.1998
(73) Proprietor: Ajinomoto Co., Inc., Tokyo 104 (JP)
(72) Inventor: Sato, Takahiro, c/o Ajinomoto Co., Inc., Kawasaki-shi, Kanagawa-ken (JP); Honda, Yutaka, c/o Ajinomoto Co., Inc., Kawasaki-shi, Kanagawa-ken (JP); Izawa, Kunisuke, c/o Ajinomoto Co., Inc., Kawasaki-shi, Kanagawa-ken (JP)
(74) Representative: Nicholls, Kathryn Margaret

(56) References cited:
- GB-A- 1 290 923
- US-A- 4 048 222
- DATABASE WPI Section Ch, Week 7723 Derwent Publications Ltd., London, GB; Class A41, AN 77-41256Y XP002042036 & SU 526 616 A (AS LITH BIOCHEM INS ET AL.) , 7 December 1976
- PAUL FRANCIS SCHUDA ET AL.: JOURNAL OF ORGANIC CHEMISTRY., vol. 53, no. 4, 1988, EASTON US, pages 873-875, XP002042034
- M. TAMURA ET AL.: SYNTHETIC COMMUNICATIONS, vol. 8, no. 5, 1978, pages 345-351, XP002042035

## Description

The present invention relates to a process for producing optically active amino acid derivatives having a cyclohexyl group which are important as intermediates of medications, especially intermediates of renin inhibitors and the like.

A cyclohexyl group-containing amino acid is a constituent of a renin inhibitor described in WO 91/07430, EP 438311, EP 427939 and Japanese Laid-Open (Kokai) No. 9,162/1993. Further, since this compound is a synthetic amino acid, it has aroused much interest from the pharmacological point of view.

A method of synthesizing a cyclohexyl group-containing amino acid is known per se. The known method synthesizes a racemic compound. Although this method uses an optically active compound as a starting material, the optical purity of the resulting amino acid is unclear, and this method is not said to include the specific production of an optically active amino acid. Nor can it be said to be an advantageous method because a solvent which can hardly be used industrially is employed and the yield is low.

Specific examples of the production of an amino acid having an optically active cyclohexyl group include the production of cyclohexylalanine by reducing L-phenylalanine in an acetic acid aqueous solution using a platinum oxide catalyst (J. Org. Chem., 1988, 53, 873, Tetrahedron, 1992, 48, 307), and the production of cyclohexylglycine by reducing (R)-phenylglycine in a carbon aqueous solution using a palladium hydroxide catalyst (Synth. Commun., 1978, 8, 345). However, in the former case, the optical purity is unclear. In the latter case, the yield is as low as between 24 and 66%, and cyclohexyl acetate is formed as a by-product at a ratio of from 27 to 68%. Further, the optical purity of the resulting cyclohexylglycine is between 66 and 84% ee, and this value is not satisfactory at all. Thus, these methods cannot be said to be industrially advantageous.

Incidentally, in the nuclear reduction of an aromatic hydrocarbon compound, the heat-reaction is generally conducted under hydrogen pressure in the presence of a catalyst such as rhodium, ruthenium, platinum or the like using an alcohol or the like as a solvent. With an unsubstituted aromatic hydrocarbon compound, the reaction proceeds relatively easily using a rhodium catalyst. However, with a hydrocarbon compound having a substituted aromatic ring, the reaction hardly proceeds owing to the influence of the resonance of the ring, or a side effect such as hydrogenolysis of a substituent or the like occurs. Thus, detailed studies on the reaction conditions are required in many cases (J. Org. Chem., 1958, 23, 276, Org. Syn., 1947, 27, 21).

With respect to reduction of an aromatic ring of an aromatic compound having a substituent with an asymmetric carbon atom, it is reported that optically active mandelic acid is reduced in the presence of a rhodium catalyst (J. Org. Chem., 1962, 27, 2288). This document describes that the racemization is suppressed almost completely. However, product crystals having a purity of 92% ee are obtained from a starting material having a purity of 95% ee, and racemization at a ratio of several percent is unavoidable. Regarding the racemization of mandelic acid, it is considered that the substituent in the benzyl position participates in the reaction as in (R)-phenylglycine described above to form a conjugated system relative to the benzyl position, with the result that a side reaction occurs along with the racemization. Consequently, the nucleic reduction of optically active phenylglycine which is conducted while maintaining the optical purity thereof is considered to be especially difficult.

Meanwhile, it is reported that a ketone group in a molecule of an L-cyclohexylalanine derivative can be reduced in an alcohol solvent in the presence of a Raney nickel catalyst (J. Org. Chem., 1988, 53, 873). However, it is described that in spite of the mild conditions, racemization occurred at a ratio of 15% simultaneously with the reduction of the ketone moiety. With some hydrogen reduction conditions it thus seems unlikely to be possible to avoid the racemization of an aromatic ring-containing amino acid.

Derwent abstract no. 77-41256y (SU-A-526616) discloses a method for producing racemic 4-amino-D,L-cyclohexyl-alanine by hydrogenation of p-nitro-D,L-phenylalanine in the presence of a ruthenium catalyst.

US-A-4 048 222 describes a process for preparing trans-4-aminomethylcyclohexane-1-carboxylic acid which comprises catalytically reducing p-aminomethylbenzoic acid in the presence of a ruthenium catalyst in a solution of a strong acid or a strong alkali as a solvent.

GB-A-1 290 923 discloses a process for producing the methyl ester of L-(β-cyclohexyl)-alanine by hydrogenation of L-tyrosine methyl ester. Platinum, palladium and rhodium are mentioned as suitable hydrogenation catalysts.

It is desirable that embodiments of the present invention provide an industrial process for producing optically active amino acid derivatives having a cyclohexyl group through the nuclear reduction of the corresponding amino acids at good yields without the racemization.

The present inventors have conducted investigations to solve the above-mentioned problems, and have consequently invented a process which may he used for producing amino acids having a cyclohexyl group at good yields without racemization by catalytically reducing aromatic amino acids in an aqueous solution containing a base, using a ruthenium catalyst.

That is, the present invention is to provide a process for producing an amino acid represented by formula (I) 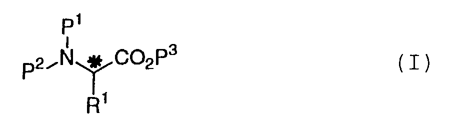 wherein
R¹ represents a cyclohexyl group or cyclohexylalkyl group having from 7 to 15 carbon atoms,
P¹ and P², independently from each other, represent a hydrogen atom or an amino-protecting group, or P¹ and P² together form a difunctional amino-protecting group,
P³ represents a hydrogen atom or a carboxyl-protecting group, and
* represents an optically active carbon atom;
   which process comprises hydrogenating an aromatic amino acid represented by formula (II) 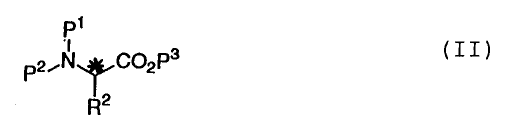 wherein
R² represents a phenyl group or an aralkyl group having from 7 to 15 carbon atoms, and P¹, P², P³ and * are as defined above,
   in the presence of a ruthenium catalyst in an aqueous or alcoholic solution containing a base. When P¹, P² and P³ in formulas (I) and (II) are all hydrogen atoms, the reaction proceeds efficiently in an aqueous solution containing at least 1 equivalent of a base.

The aromatic amino acid represented by formula (II) as used in the present invention may be natural or synthetic optically active amino acids.

Examples of the aromatic amino acid include L-phenylalanine, D-phenylalanine, D-phenylglycine, L-phenylglycine, L-tyrosine and D-tyrosine.

Examples of the aromatic amino acid derivatives include N-protected amino acids obtained by protecting the amino group of the above-mentioned aromatic amino acids, amino acid esters obtained by protecting the carboxyl group thereof, and N-protected amino acid esters obtained by protecting the amino group and the carboxyl group thereof. Any N-protecting group which is ordinarily used in peptide synthesis is available. Examples thereof include a tert-butoxycarbonyl group, an acetyl group, a formyl group, a trifluoroacetyl group and a phthaloyl group. Any carboxyl-protecting group which is ordinarily used in the peptide synthesis is available. Examples thereof include an ethyl ester group, a methyl ester group and a tert-butyl ester group. Further, examples of the N-protecting group include a benzyloxycarbonyl group and a dibenzyl group. Examples of the carboxyl-protecting group include a dibenzyl ester group. Thus, these protecting groups which are deprotected through reduction can also be used. In this case, a deprotected product is provided.

The protected aromatic amino acid derivatives can easily be synthesized by an ordinary production method.

When the starting material is an unprotected amino acid, the reaction may easily proceed through stirring in an aqueous solution containing at least 1 equivalent of a base under hydrogen pressure in the presence of a ruthenium catalyst.

On the other hand, when the starting material is an N-protected amino acid, the reaction may easily proceed through stirring in an aqueous solution or an alcohol solution (eg a solution in ethanol) at a hydrogen pressure in the presence of a ruthenium catalyst. In this case, the reaction also preferably proceeds in at least 1 equivalent of a base.

Specifically, from 1- to 10-% ruthenium on carbon may be used as a ruthenium catalyst in an amount of from 0.001 to 0.1 equivalents based on the amount of the starting amino acid, and a hydrogen pressure of from 1 to 100 atm (1.01 × 10⁵ - 1.01 × 10⁷ N/m²) may preferably be employed.

Examples of the base include sodium hydroxide, potassium hydroxide and ammonia. The amount of the base is between 1 and 10 equivalents, preferably between 1 and 2 equivalents. The concentration of the base is, for example, between 0.1 and 2.0 N.

Preferred reaction temperature is between room temperature and 250°C, particularly preferably between 40 and 150°C. The reaction may be completed in from 1 to 24 hours.

The reaction solution may be filtered to remove the catalyst. Subsequently, eg if the reaction was conducted in a basic aqueous solution, the residue may be neutralized or acidified to precipitate the product in the form of a free substance or a salt. Thus, the final product can easily be isolated and purified.

### Examples

The present invention is illustrated more specifically by referring to the following Examples. However, the present invention is not limited thereto. The temperature is indicated in terms of a centigrade temperature unless otherwise indicated. Proton nucleic magnetic resonance spectra were recorded on a varian 300 MHz spectrometer; chemical shift (δ)is shown below in terms of ppm. The analysis of N-unprotected amino acid optical isomers was conducted using a Shimadzu Optical Analysis Gas Chromatography DLAA-1 (column: Chirasil Val/ASA) System. The isomers were determined from areas (%) on a chart given by automatic derivatization into N-trifluoroacetyl and isopropyl esters and analysis thereof.

### Example 1

### Production of L-cyclohexylalanine·hydrochloride:

L-phenylalanine (2.010 g, 12.2 mmols) and 105.1 mg (0.05 mmols) of 5-% ruthenium on active carbon as a catalyst were dissolved in 15 ml of a 1-N sodium hydroxide aqueous solution, and the solution was stirred at 60°C and a hydrogen pressure of 30 kg/cm² (2.94 × 10⁶ N/m²) for 4.5 hours. The analysis of the reaction solution through HPLC revealed that the reduction proceeded quantitatively. With respect to the optical purity at this time, the area ratio was found to be L:D = 99.5 : 0.5 as a result of the analysis through optical analysis gas chromatography. After the completion of the reaction, the reaction mixture was filtered through Celite to remove the ruthenium on active carbon as a catalyst. Thus, a sodium hydroxide solution of cyclohexylalanine was obtained. The resulting filtrate was concentrated to approximately 1/3 of the original volume under reduced pressure. Then, 11 ml (66 mmols) of a 6-N hydrochloric acid aqueous solution and 12.8 ml of water were added to the residue. The mixture was dissolved while being heat-stirred, and then precipitated through cooling to give 1.681 g (98.3% by weight, 7.96 mmols) of L-cyclohexylalanine hydrochloride in a yield of 65.2%.

As a result of the analysis through optical analysis gas chromatography, the area ratio was found to be L:D = 99.7 : 0.3.
1H-NMR(D2O) δ :0.88-1.08(m,2H),1.15-1.34(m,3H),1.34-1.52 (m, 1H),1.58-1.86(m,7H),3.89(dd,1H)

### Example 2

### Production of (S)-cyclohexylglycine·hydrochloride:

(S)-phenylglycine (1.966 g, 13.0 mmols) and 106.6 mg (0.05 mmols) of 5-% ruthenium on active carbon as a catalyst were dissolved in 15 ml of a 1-N sodium hydroxide aqueous solution. The solution was then stirred at 60°C and a hydrogen pressure of 30 kg/cm² (2.94 × 10⁶ N/m²) for 5 hours. The analysis of the reaction solution through HPLC revealed that the reduction proceeded quantitatively. At this time, as a result of the analysis through optical analysis gas chromatography, the area ratio was found to be S:R = 99.2 : 0.8. After the completion of the reaction, the reaction mixture was filtered through a Celite to remove the ruthenium on active carbon as a catalyst and obtain a sodium hydroxide solution of cyclohexylglycine. The resulting filtrate was concentrated to approximately 1/3 of the original volume under reduced pressure, and 11.2 ml (67.2 mmols) of a 6-N hydrochloric acid aqueous solution and 1 ml of water were added to the residue. The mixture was dissolved while being heat-stirred, and was precipitated through cooling to give 1.678 g (100% by weight, 8.7 mmols) of (S)-cyclohexylglycine·hydrochloride in a yield of 65.4%. As a result of analysis through optical analysis gas chromatography, the area ratio was found to be S = > 99.98 (R = < 0.02).
1H-NMR(D2O) δ:1.05-1.37(m,5H) 1.62-1.81(m,5H) 1.93-2.03(m,1 H) 3.82(d,1H)

## Claims

1. A process for producing an amino acid represented by formula (I) 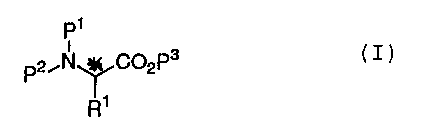 wherein
R¹ represents a cyclohexyl group, or a cyclohexylalkyl group having from 7 to 15 carbon atoms
P¹ and P², independently from each other, represent a hydrogen atom or an amino-protecting group, or P¹ and P² together form a difunctional amino-protecting group,
P³ represents a hydrogen atom or a carboxyl-protecting group, and
* represents an optically active carbon atom;
which process comprises hydrogenating an aromatic amino acid represented by formula (II) 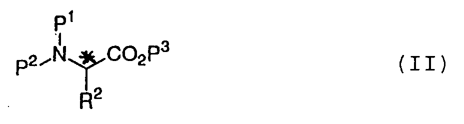 wherein:
R² represents a phenyl group or an aralkyl group having from 7 to 15 carbon atoms, and
P¹, P², P³ and * are as defined above;
wherein the hydrogenation is carried out in the presence of a ruthenium catalysts and in an aqueous or alcoholic solution containing a base.

2. The process of claim 1, wherein P³ in formulas (I) and (II) is hydrogen atom.

3. The process of claim 1 or claim 2, wherein P¹, P² and P³ in formulas (I) and (II) are all hydrogen atoms.

4. The process of any one of the preceding claims, wherein the reaction is conducted in an aqueous solution containing at least 1 equivalent of a base.

5. The process of any one of claims 1 to 4, wherein the optically active carbon atom in formulas (I) and (II) has an S-configuration.

6. The process of any one of claims 1 to 4, wherein the optically active carbon atom in formulas (I) and (II) has an R- configuration.

## Patentansprüche

1. Verfahren zur Herstellung einer durch Formel (I) dargestellten Aminosäure 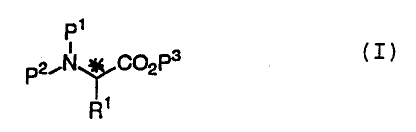
worin R¹ eine Cyclohexylgruppe oder eine Cyclohexylalkylgruppe mit 7 bis 15 Kohlenstoffatomen darstellt,
P¹ und P² unabhängig voneinander ein Wasserstoffatom oder eine Amino-Schutzgruppe darstellen oder P¹ und P² zusammen eine difunktionelle Amino-Schutzgruppe darstellen,
P³ ein Wasserstoffatom oder eine Carboxyl-Schutzgruppe darstellt und
* ein optisch aktives Kohlenstoffatom anzeigt;
wobei das Verfahren die Hydrierung einer durch Formel (II) dargestellten aromatischen Aminosäure 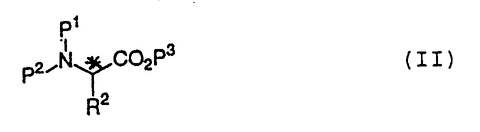 umfaßt,
worin R² eine Phenylgruppe oder eine Aralkylgruppe mit 7 bis 15 Kohlenstoffatomen darstellt und
P¹, P², P³ und * die vorstehende Definition haben,
wobei die Hydrierung in Gegenwart eines Rutheniumkatalysators und in einer eine Base enthaltenden wäßrigen oder alkoholischen Lösung durchgeführt wird.

2. Verfahren nach Anspruch 1, wobei P³ in den Formeln (I) und (II) ein Wasserstoffatom ist.

3. Verfahren nach Anspruch 1 oder Anspruch 2, wobei alle Symbole P¹, P² und P³ in den Formeln (I) und (II) Wasserstoffatome sind.

4. Verfahren nach einem der vorhergehenden Ansprüche, wobei die Reaktion in einer wäßrigen Lösung durchgeführt wird, die mindestens ein Äquivalent einer Base enthält.

5. Verfahren nach einem der Ansprüche 1 bis 4, wobei das optisch aktive Kohlenstoffatom in den Formeln (I) und (II) eine S-Konfiguration hat.

6. Verfahren nach einem der Ansprüche 1 bis 4, wobei das optisch aktive Kohlenstoffatom in den Formeln (I) und (II) eine R-Konfiguration hat.

## Revendications

1. Procédé de préparation d'un acide aminé représenté par la formule (I) 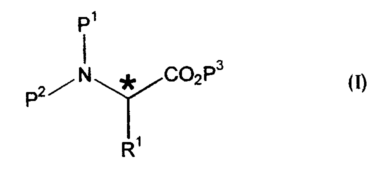 dans laquelle
R¹ représente un groupe cyclohexyle ou un groupe cyclohexylalkyle ayant de 7 à 15 atomes de carbone,
P¹ et P², indépendamment l'un de l'autre, représentent un atome d'hydrogène ou un groupe protecteur de groupe amino, ou P¹ et P² forment ensemble un groupe protecteur de groupe amino difonctionnel,
P³ représente un atome d'hydrogène ou un groupe protecteur de groupe carboxyle, et
* représente un atome de carbone actif optiquement,
ce procédé comprenant l'hydrogénation d'un acide aminé aromatique représenté par la formule (II) 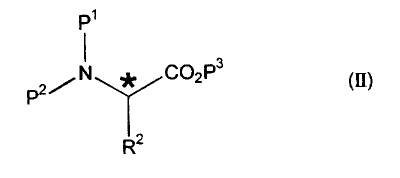 dans laquelle
R² représente un groupe phényle ou un groupe aralkyle ayant de 7 à 15 atomes de carbone, et
P¹, P², P³ et * sont tels que définis ci-dessus, dans lequel l'hydrogénation est effectuée en présence d'un catalyseur de ruthénium et dans une solution aqueuse ou alcoolique contenant une base.

2. Procédé selon la revendication 1, caractérisé en ce que P¹ dans les formules (I) et (II) est un atome d'hydrogène.

3. Procédé selon la revendication 1 ou la revendication 2, caractérisé en ce que P¹, P² et P³ dans les formules (I) et (II) sont tous des atomes d'hydrogène.

4. Procédé selon l'une quelconque des revendications précédentes, caractérisé en ce que la réaction est effectuée dans une solution aqueuse contenant au moins 1 équivalent d'une base.

5. Procédé selon l'une quelconque des revendications 1 à 4, caractérisé en ce que l'atome de carbone actif optiquement dans les formules (I) et (II) a une configuration (S).

6. Procédé selon l'une quelconque des revendications 1 à 4, caractérisé en ce que l'atome de carbone actif optiquement dans les formules (I) et (II) a une configuration R.
